# EUROPEAN PATENT SPECIFICATION

(11) **EP 2 882 729 B1**
(45) Date of publication and mention of the grant of the patent: **21.09.2016**
(21) Application number: 13748030.7
(22) Date of filing: 12.08.2013
(51) Int. Cl.: C07D 311/72

(54) **PROTECTED TOCOPHEROL WITH REDUCED CONTENT OF FREE TOCOPHEROL**
GESCHÜTZTES TOCOPHEROL MIT REDUZIERTEM ANTEIL AN FREIEN TOCOPHEROLEN
TOCOPHÉROL PROTÉGÉ AYANT UN CONTENU RÉDUIT EN TOCOPHÉROL LIBRE

(30) Priority: 10.08.2012 EP 12180085
(43) Date of publication of application: 17.06.2015
(73) Proprietor: DSM IP Assets B.V., 6411 TE Heerlen (NL)
(72) Inventor: WILDERMANN, Angela, CH-4002 Basel (CH)
(74) Representative: Dux, Roland
(86) International application number: PCT/EP2013/066819
(87) International publication number: WO 2014/023848

(56) References cited:
- WO-A1-91/17985
- WO-A2-2005/054223
- WO-A2-2006/018310
- FR-A- 962 797
- US-A1- 2002 017 451
- ROSSI M ET AL: "The effect of bleaching and physical refining on color and minor components of palm oil", JOURNAL OF THE AMERICAN OIL CHEMISTS' SOCIETY, vol. 78, no. 10, October 2001 (2001-10), pages 1051-1055, XP002689679, ISSN: 0003-021X
- KEITO BOKI: "BLEACHING OF ALKALI-REFINED VEGETABLE OILS WITH CLAY MINERALS", JOURNAL OF THE AMERICAN OIL CHEMISTS' SOCIETY, SPRINGER, DE, vol. 69, no. 3, 1 March 1992 (1992-03-01), pages 232-236, XP000245388, ISSN: 0003-021X, DOI: 10.1007/BF02635892
- SABA NAZ ET AL: "Changes of Total Tocopherol and Tocopherol Species During Sunflower Oil Processing", JOURNAL OF THE AMERICAN OIL CHEMISTS' SOCIETY, vol. 88, no. 1, 1 January 2011 (2011-01-01), pages 127-132, XP055048035, ISSN: 0003-021X, DOI: 10.1007/s11746-010-1652-4 cited in the application

## Description

### Technical Field

The invention relates to the field of preparation and use of protected tocopherols.

### Background of the invention

Tocopherols are important substances in the field of food and feed ingredients. It is already known for a long time that tocopherols are part of vitamin E and show an important bioactivity. In order to increase the stability of tocopherols the phenolic hydroxyl group can be protected, particularly as acetate. The protected tocopherol can be easily produced from the corresponding tocopherols and deprotected again easily into the tocopherol on demand, for example in a body of an animal or a human being. It is known that protected tocopherols comprise small amount of impurities. One important impurity is free tocopherol. The European Pharmacopoeia, 5th edition, 2005, Supplement 5.3, page 3632 - 3633 defines this impurity as impurity C for *(all-rac)*-α-tocopheryl acetate. The maximum limit for this impurity defined therein is 0.5 % in *(all*-*rac)*-a-tocopheryl acetate. It has been observed that particularly additional thermal stress of the protected tocopherols such as occurring in rectification processes leads to increased levels of free tocopherols. It is therefore desirable to have a concentration below said limit, and to lower the level of impurity further, respectively even to eliminate this impurity completely.

Vegetable oils and fats often have undesired strong odors and colors and, hence, refinement is necessary to have an acceptable quality for the consumption by animals or humans. This refinement involves different purification steps of which bleaching is the most prominent step. It is known since a long time that bleaching earths are very efficient in the refinement of edible oils and fats. Bleaching earths are also known as bleaching clays. It was found that for certain purposes, bleaching earths may be modified by the treatment with strong acids and that those acid-treated bleaching earths, which are also known as acid activated bleaching earths, exhibit a better performance in the bleaching. However, it is known, for example from J. Am. Oil Chem. Soc (2011) 88: 127-132, that the level of tocopherols being present in the oil to be refined is remarkably reduced by the bleaching step and that the adsorption of the tocopherols on the bleaching clays is regarded as reason for this. Therefore, tocopherols are often added to edible oils and fats to increase the level of tocopherols again in order to stabilize those oils against further oxidative deteriorations such as disclosed in US 4,101,673.

FR 962 797 discloses that tocopherols can be obtained from different vegetable oils. It further discloses a method of purifying and/or separation of tocopherols from such sources by means of an adsorbent being clay, kaolin or silica gel. In this method first the tocopherol is adsorbed from a highly diluted solution in a non-polar solvent onto the adsorbent, preferably in a column, and, hence, is separated from the rest of the sourcing composition. In a second step the adsorbed tocopherol is then extracted from the adsorbent by percolating by means of another solvent being more polar. By using different solvents alpha and gamma tocopherols can be separated from each other and isolated from a mixture or concentrate as they result from natural vegetable oil sources. However, this purification is very expensive as it requires a large amount of adsorbent as well as of different solvents and is not suitable for being used for large quantities and highly concentrated forms of tocopherols as they result from chemical synthesis.

WO 2006/018310 discloses a process for the preparation of acetylated tocopherol.

The need for protected tocopherol on the world's market is extremely large, and, therefore, the major part of commercially available tocopherols is synthesized from petrochemicals and is not originating from bio-sources. These syntheses yield tocopherol in neat form or in very highly concentrated solutions.

### Summary of the invention

The problem to be solved by present the invention is to reduce the level of the impurity of tocopherol of formula (II) in the protected tocopherol of formula (I) as defined below.

Surprisingly, it has been found that the method according to claim 1 is able to solve this problem. Particularly contacting the tocopherol used for its preparation with acid activated bleaching earth leads to a remarkably reduction of said impurity. It has been observed that this reduction of said impurity is particularly significant in cases where the protected tocopherols are submitted to additional thermal stresses, such as occurring when the protected tocopherols are rectified with the goal to obtain highly purified protected tocopherols.

It is particularly surprising that exactly acid activated bleaching earths are suitable for this purpose in consideration of the fact that it is known that such adsorbing material exactly adsorbs in preference the product to be treated, i.e. the tocopherols.

The method is very efficient and easy and allows producing protected tocopherols (e.g. tocopheryl acetates) in a very high quality and purity, thus, this method is very attractive to producers of vitamin E and to the market. It was particularly found that the amount of said impurity, the tocopherol of formula (II), can be reduced by more than 30% by weight, preferably by more than 40 % by weight, and even more preferably by more than 45 % by weight. Particularly, it has been found that the amount of said impurity can be reduced below 0.5 %, particularly below 0.25 %, more particularly below 0.20 % by weight, based on the weight of the protected tocopherol of formula (I).

Further aspects of the invention are subject of further independent claims. Particularly preferred embodiments are subject of dependent claims.

### Detailed description of the invention

The present invention relates in a first aspect to a method of preparing a protected tocopherol of formula (I).
comprising the steps of
a) contacting tocopherol of formula (II), in the form of neat tocopherol of formula (II) or in the form of a solution comprising more than 60 % by weight of tocopherol of formula (II), with an acid activated bleaching earth;
   and
b) reacting tocopherol of formula (II) after contacting step a) with a protecting agent to yield protected tocopherol of formula (I); 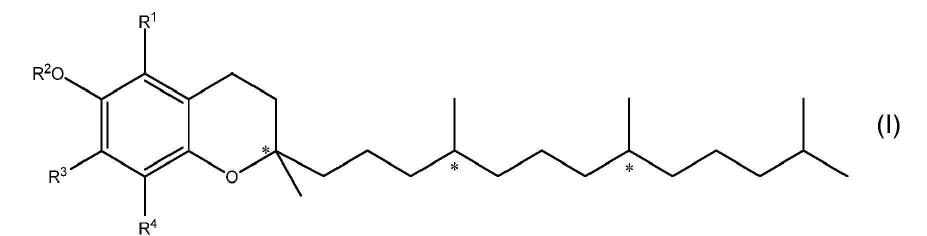 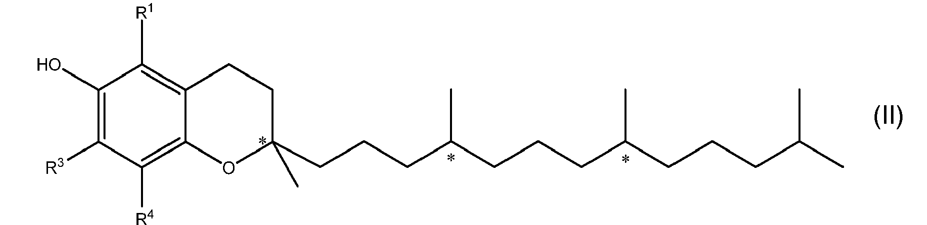 wherein R¹, R³ and R⁴ are independently from each other hydrogen or methyl groups;
   R² represents a phenol protection group;
   and wherein each * represents an individual chiral centre.

The term "independently from each other" in this document means, in the context of substituents, moieties, or groups, that identically designated substituents, moieties, or groups can occur simultaneously with a different meaning in the same molecule.

A "C_{x-y}-alkyl" or "C_{x-y}-acyl" group is an alkyl or an acyl group, respectively, comprising x to y carbon atoms, i.e., for example a C₁₋₃-alkyl group is an alkyl group comprising 1 to 3 carbon atoms. The alkyl group or the acyl group can be linear or branched.

The term "hydrogen" means in the present document H and not H₂.

R² represents a phenol protection group. A phenol protection group is a group which protects the phenolic group in formula (I) and can be deprotected easily, i.e. by state-of-the-art methods, to the phenolic group again.

The tocopherol of formula (II) carries the same groups R¹, R³ and R⁴ as the protected tocopherol of formula (I). For example, if formula (I) has the groups R¹ = R⁴ = CH₃, R³ = H, then also formula (II) has the groups R¹ = R⁴ = CH₃, R³ = H.

The chirality at the individual chiral centres indicated by * of the protected tocopherol of formula (I) correspond to the chirality at the individual corresponding chiral centres of the tocopherol of formula (II).

The phenol protection group forms with the rest of the molecule a chemical functionality which is selected from the group consisting of ester, ether or acetal. The protection group can be easily removed by standard methods known to the person skilled in the art.

In case where the phenol protection group forms with the rest of the molecule an ether, the substituent R² is particularly a linear or branched C₁₋₁₀-alkyl or cycloalkyl or aralkyl group. Preferably the substituent R² is a benzyl group or a substituted benzyl group, particularly preferred a benzyl group.

In case where the phenol protection group forms with the rest of the molecule an ester, the ester is an ester of an organic or inorganic acid.

If the ester is an ester of an organic acid, the organic acid can be a monocarboxylic acid or a polycarboxylic acid, i.e. an acid having two or more COOH-groups. Polycarboxylic acids are preferably malonic acid, succinic acid, glutaric acid, adipic acid, maleic acid or fumaric acid.

Preferably the organic acid is a monocarboxylic acid.

Hence, the substituent R² is preferably an acyl group. The acyl group is particularly a C₁₋₇-acyl, preferably acetyl, propionyl or benzoyl group, or a substituted benzoyl group.

If the ester is an ester of an inorganic acid, the inorganic acid is preferably nitric acid or a polyprotic acid, i.e. an acid able to donate more than one proton per acid molecule, particularly selected from the group consisting of phosphoric acid, pyrophosphoric acid (=diphosphoric acid), phosphorous acid (=phosphonic acid), sulphuric acid and sulphurous acid.

In case where the phenol protection group forms with the rest of the molecule an acetal, the substituent R² is preferably 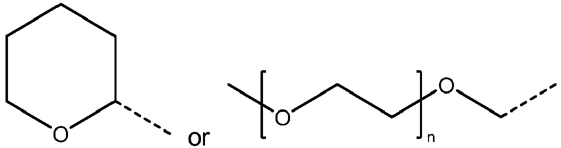 with n=0 or 1.

Hence, the acetals formed are preferably methoxymethyl ether (MOM-ether), β-methoxyethoxymethyl ether (MEM-ether) or tetrahydropyranyl ether (THP-ether). The protection group can easily be removed by acid.

In the present document any dotted line represents the bond by which a substituent is bound to the rest of a molecule.

The tocopherol of formula (II) is reacted in step b) with a protecting agent to yield a protected tocopherol of formula (I).

The protecting agents leading to the corresponding phenol protection groups are known to the person skilled in the art, as well as the chemical process and conditions for this reaction. If, for example, the phenol protection group forms with the rest of the molecule an ester, the suitable protecting agent is for example an acid, an anhydride or an acyl halide.

In the case that an ester is formed by the reaction of tocopherol of formula (II) with a protecting agent, and that said ester is an ester of an organic polycarboxylic acid or an inorganic polyprotic acid, not necessarily all acid groups are esterified to qualify as protected tocopherols of formula (I) in the sense of this document. Preferable esters of inorganic polyprotic acids, corresponding to protected tocopherols of formula (I), are tocopheryl phosphates and ditocopheryl phosphates, particularly α-tocopheryl phosphate and α-ditocopheryl phosphate.

It is preferred that the protection group is a benzoyl group or a C₁₋₄-acyl group, particularly acetyl group. The molecules in which R² represents an acyl group, particularly an acetyl group, can be easily prepared from the corresponding tocopherol of formula (II) by esterification, conversely, the phenolic compound can be obtained from the corresponding ester by ester hydrolysis.

The protected tocopherols are additionally rectified after the protection reaction step (i.e. step b). This method is particularly used for the production of highly purified protected tocopherols, particularly "pharma grade" protected tocopherols. In the present document the term "highly purified protected tocopherols" means that purity of protected tocopherols is more than 96.5 % by weight. As protected tocopherols have very high boiling temperatures, the thermal stress involved in a rectification process is substantial. The temperatures measured in the sump are in the range of 220- 280 °C.

The tocopherol of formula (II), in the form of neat tocopherol of formula (II) or in the form of a solution comprising more than 60 % by weight of tocopherol of formula (II), is contacted in step a) with an acid activated bleaching earth, i.e. the step of contacting with the acid activated bleaching earth occurs before the protection reaction step (i.e. step b) in the preparation of protected tocopherol of formula (I).

### Acid activated bleaching earth

The term "bleaching earth" (German: "Bleicherde") is known to the person skilled in the art to be a collective name for a group of aluminium and/or magnesium silicates comprising water (Römpp, Lexikon der Chemie, Bleicherde, 10. Auflage, Stuttgart, 1996, page 469). Particularly suitable bleaching earths are attapulgite, bentonite and montmorillonite.

"Acid activated bleaching earths" are bleaching earths which are treated by strong mineral acids. This acid treatment leads to ion exchange in the bleaching earth. Due to this ion-exchange, i.e. acid activation, the properties of the bleaching earths are strongly modified.

The acid activated bleaching earth is preferably an acid activated bentonite. Particularly suitable are acid activated bleaching earths which are commercialized under the trademark TONSIL® by Süd-Chemie.

In a further embodiment the acid activated bleaching earths are those commercialized as activated clays F-20 and F-20X by BASF.

The acid activated bleaching earth has preferably a specific surface area (BET) between 150 and 400 m²/g, preferably between 250 and 375 m²/g, more preferably between 300 and 350 m²/g. The specific surface area is measured according to the method BET (Brunauer, Emmett und Teller) which is a commonly known method for determination of surface areas of solid particles by physical adsorption of gas, particularly as described in ISO 9277.

Particularly a 10 % by weight suspension in water has a pH of between 1.5 and 5, preferably between 1.5 and 4, measured at 25°C and after filtration.

The acidity of the acid activated bleaching earth, measured as mg KOH/g, is preferably in the range of 0.1 to 22, more preferably in the range of 0.2 to 21, even more preferably in the range of 0.3 to 20.

Furthermore, it is preferred that the acid activated bleaching earth has after drying at 110°C during 2 hours a residual amount of CaO of less than 1.5 % by weight, particularly between 0.001 and 0.8 % by weight.

Particularly suitable are the products of the trademark TONSIL® SUPREME such as TONSIL® SUPREME 110 FF, TONSIL® SUPREME 112 FF, TONSIL® SUPREME 114 FF, TONSIL® SUPREME 115 FF and TONSIL® SUPREME 118 FF and TONSIL® Optimum 210 FF allowing a fast separation, particularly a fast filtration. Furthermore suitable are the products sold as activated clays F-20 and F-20X by BASF. It has been observed that TONSIL® SUPREME 115 FF, activated clay F-20, and F-20X are particularly suitable for the purpose of the present invention.

Acid activated bleaching earths are very interesting in view of toxicological aspects. Acid activated bleaching earths are particularly suitable for the treatment of food and feed ingredients, since for a long time already, acid activated bleaching earths are used on an industrial scale for the treatment of vegetable oils and fats. The acid activated bleaching earths can also be easily removed completely from the tocopherols.

As mentioned above the protected tocopherol of formula (I) is formed from tocopherol of formula (II) by a reaction in step b) with a protecting agent.

Tocopherol of formula (II) used for this protection reaction is a known product as such and is part of vitamin E. It can be produced in different ways as disclosed in Ullmann's Encyclopedia of Industrial Chemistry, Release 2010, 7th Edition, "Vitamins", pages 43 - 47 or in WO 2005/054223 A2.

The preferred route of synthesis is the condensation of the corresponding methyl-, dimethyl- or trimethylhydoquinone of formula (III) with phytol or isophytol.

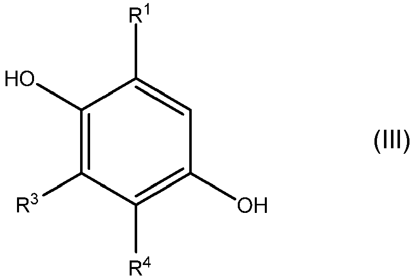

For this condensation a series of catalysts may be used such as ZnCl₂/mineral acid, BF₃/AlCl₃, Fe/HCl, trifluoroacetic acid or boric acid/carboxylic acid as well as indium (III) or scandium (III) salts as disclosed in WO 2005/121115 A1. Furthermore suitable catalysts are heteropoly acids, particularly 12-tungstophosphoric or 12-tungstosilicic acid.

Said reactions are not stereospecific and, hence, a mixture of isomers of tocopherol of formula (II) is obtained. This is reflected by the use of the * in the formulae of this document. Due to the fact that there are 3 chiral centres in compounds of formula (I) or (II) there exist 8 individual isomers. Such a product being a mixture of R- and S- configuration at each chiral centre (2, 4' and 8') indicated by * in this document is also called (*all*-*rac*)-tocopherol or (*all*-*rac*)-tocopheryl acetate (in case the phenol protection group is an acetyl group).

In view of industrial interest and volume of production it is preferred that tocopherol of formula (II) is (*all*-*rac*)-tocopherol, particularly (*all*-*rac*)--α-tocopherol and that the protected tocopherol of formula (I) is (*all*-*rac*)-tocopheryl acetate, particularly (*all*-*rac*)-α-tocopheryl acetate.

Since the different isomers have different bioactivity it is in the interest to synthesize specifically a desired isomer having high bioactivity. (2R, 4'R, 8'R)-tocopherol isomers have shown to exhibit a particularly high bioactivity. There exist such stereoselective syntheses which yield to either specific isomers or mixtures with less isomers only. For example, natural phytol can be used for the above synthesis. Natural phytol consists only of the R,R-isomer and, hence, is isomerically pure. Therefore, the above reaction leads to a mixture of (2R, 4'R, 8'R)-tocopherol and (2S, 4'R, 8'R)-tocopherol, also known as 2-*ambo*-tocopherol. However, as natural phytol or isophytol is commercially available only in rather small amounts and is rather expensive, the potential of using natural phytol or isophytol for industrial scale synthesis of tocopherols is rather limited.

Is has been found, however, that by using specific chiral iridium complexes, asymmetrical hydrogenation of alkenes can be achieved. The chiral iridium complexes disclosed in WO 2006/066863 A1 as well as in the pending application EP11169784.3 are particularly suited for this purpose. By this use of asymmetrical hydrogenation 2-*ambo-*tocopherol can be obtained, the isomers of which can be separated much more easily, more efficiently and particularly in a much more cost effective way than isomeric mixtures obtained by traditional processes.

Therefore, it is preferred in another embodiment that the tocopherol of formula (II) is a mixture of (2R, 4'R, 8'R)-tocopherol and (2S, 4'R, 8'R)-tocopherol or that the protected tocopherol of formula (I) is a mixture of (2R, 4'R, 8'R)-tocopheryl acetate and (2S, 4'R, 8'R)-tocopheryl acetate.

More preferred, the tocopherol of formula (II) is (2R, 4'R, 8'R)-tocopherol or the protected tocopherol of formula (I) is (2R, 4'R, 8'R)-tocopheryl acetate.

Most preferred, the protected tocopherol of formula (I) is (2R, 4'R, 8'R)-α-tocopheryl acetate and the tocopherol of formula (II) is (2R, 4'R, 8'R)-α-tocopherol.

Preferred are the following combinations of R¹, R³ and R⁴:

R¹ = R³ = R⁴ = CH₃

or

R¹ = R⁴ = CH₃, R³ = H

or

R¹ = H, R³ = R⁴ = CH₃

or

R¹ = R³ = H, R⁴ = CH₃

Most preferred is the combination R¹ = R³ = R⁴ = CH₃.

Therefore, preferably the protected tocopherol of formula (I) is selected from the group consisting of α-tocopheryl acetate, β-tocopheryl acetate, γ-tocopheryl acetate and δ-tocopheryl acetate, particularly α-tocopheryl acetate.

It is preferred that only one type of tocopherol of formula (II), is present when the contacting with the acid activated bleaching earth is performed. In other words it is preferred that no mixtures of different tocopherols with different groups R¹, R³ and R⁴, particularly no mixtures comprising α-tocopherol and γ-tocopherol, are contacted with acid activated bleaching earth.

A key element of the present invention is the interaction of acid activated bleaching earth with the tocopherol of formula (II), in the form of neat tocopherol of formula (II) or in the form of a solution comprising more than 60 % by weight of tocopherol of formula (II), in step a). This interaction requires a physical contact of the tocopherol with the acid activated bleaching earth. It is desired that the contact is thorough. Hence, any possibility allowing a thorough contact between the surface of the acid activated bleaching earth with the tocopherol of formula (II) can be principally used.

A preferred possibility for such a thorough contact, which is particularly suitable for the purpose of the invention, is when the contacting is realized by adding in step a) the acid activated bleaching earth to the tocopherol of formula (II) and by stirring.

This leads to a suspension of acid activated bleaching earth in a liquid phase comprising the tocopherol of formula (II). This can be realized in a continuous or in a batchwise manner in a stirred tank or loop reactor or a cascade of those reactor types.

There exist also other possibilities of contacting. The contact with the acid activated bleaching earth can be realized for example by a flow of the tocopherol over the acid activated bleaching earth being immobilized on a carrier such as a plate or a wall or by a flow through a column filled with acid activated bleaching earth. In order to increase the time of contact between activated bleaching earth and the tocopherol it is advisable to use a flow in a continuous loop. However, it has been observed that particularly the use of a fixed bed reactor is disadvantageous as the contact is not sufficient enough to allow an industrially effective process at an acceptable pressure drop.

It is preferred that the contacting with acid activated bleaching earth is performed under inert conditions, particularly under nitrogen.

Furthermore, it has been shown that the contact is particularly efficient when the contacting is done at elevated temperature, particularly at a temperature of between 80 and 160 °C, preferably between 100 and 150°C.

It is further preferred that the time of contact is between 30 and 300 minutes, preferably between 45 and 180 minutes, more preferably between 60 and 120 minutes.

Particularly preferred is when the contact is realized at an absolute pressure of 0.01 - 1013 mbar, preferably 10 - 500 mbar, more preferably 20 - 200 mbar.

The step of contacting can be realized in a continuous or in a batch process.

It has been observed that it is preferred that the weight ratio of acid activated bleaching earth to tocopherol of formula (II) in step a), is between 0.5 / 100 and 5 / 100, particularly is between 1 / 100 and 3 / 100, preferably between 1 / 100 and 2 / 100. For example, using 5 g acid activated bleaching earth per 100 g tocopherol of formula (II) leads to a weight ratio of acid activated bleaching earth to tocopherol of formula (II) of 5 / 100.

The tocopherol of formula (II) is contacted as such, i.e. in the form of neat tocopherol of formula (II), or in the form of a solution comprising more than 60 %, preferably more than 90 %, particularly more than 95 %, by weight of tocopherol of formula (II), with the acid activated bleaching earth. For the solution a non-polar solvent, particularly selected from the group consisting of hexane, heptane, xylene and toluene is preferred.

The use of solvents to yield a solution is advantageous in view of lowering the viscosity of the tocopherol of formula (II). A lower viscosity enables a better contact, particularly at lower temperatures and an easier and faster separation from the acid activated bleaching earth.

As the method of invention focuses particularly to tocopherols originating from chemical synthetic processes, any solution of the tocopherol of formula (II) should also not comprise any triglycerides or any fatty acids, particularly as they occur in vegetable oils.

After contacting of the tocopherol of formula (II) with an acid activated bleaching earth the acid activated bleaching earth is separated from the tocopherol of formula (II) in a further step. The separation can be achieved for example by filtering off the acid activated bleaching earth after the contacting or by centrifugation.

### Reduction of amount of tocopherol of formula (II)

The present invention leads to protected tocopherol of formula (I) with a reduced content of tocopherol of formula (II).

The amount of tocopherol of formula (II) in protected tocopherol of formula (I) is reduced at least by 30 %, particularly at least 40 %, preferably at least 45 %, by weight compared to the amount of the corresponding tocopherol of formula (II) measured in protected tocopherol of formula (I) for the preparation of which no step a) of contacting with acid activated bleaching earth has been used ("*comparative protected tocopherol*").

It is important to stress that this definition relies on the comparison of two protected tocopherols of formula (I) being synthesized and treated in a completely identical manner except that the step a) of contacting tocopherol of formula (II) with an acid activated bleaching earth has not been performed for the *comparative protected tocopherol.*

It has been observed that a variety of parameters have a more or less pronounced effect on the degree of reduction of the amount of tocopherol of formula (II) found in the protected tocopherol of formula (I), such as temperature, pressure, time of contacting or origin of starting materials.

The amount of the tocopherol of formula (II) can be determined by gas chromatography.

Particularly the amount of tocopherol of formula (II) is measured by means of a gas chromatograph (Agilent, model 6850) using an autosampler, a FID detector and a column HP Ultra 2 (Crosslinked 5 % phenylsilanol, 95 % methylsilanol; length 25 m, 0.32 mm internal diameter, 0.52 µm film) and the following parameters: carrier gas: H₂, constant flow: 2.5ml/min, injector temperature: 280°C, injection volume: 1.0 µl, temperature program: 180°C to 280°C rate 20.0 °C/min, detector temperature: 300°C. The sample was injected as a solution of 4 mg/ml in heptane.

In case of α-tocopheryl acetate, the peak at a retention time of 13.1 minutes was identified to be α-tocopherol.

The amount was determined by calibration with an authentic reference substance to be detected, such α-tocopherol.

With the method of invention, a residual amount of tocopherol of formula (II) of < 0.5 % by weight, particularly < 0.25 % by weight, based on the weight of protected tocopherol of formula (I) can be easily achieved. Within the range indicated above, by using a higher weight ratio of acid activated bleaching earth to tocopherol of formula (II) in step a), it has been found that the residual content of tocopherol of formula (II) in the protected tocopherol of formula (I) after the protection reaction in step b), particularly after any additional thermal stress, e.g. by rectification, of the protected tocopherol of formula (I), is even below 0.20 %. By further optimizing the present method, it is possible to reduce the amount of this impurity even further, so that the residual amount of tocopherol of formula (II) in protected tocopherol of formula (I) is even below 0.05 %.

In a preferred embodiment, a residual amount of *(all*-*rac)*-α-tocopherol in the *(all*-*rac)*-α-tocopheryl acetate after the acetylation reaction in step b), particularly after the rectification of *(all*-*rac)*-α-tocopheryl acetate, of < 0.5 % by weight, particularly < 0.25 % by weight, preferred < 0.20 % by weight, based on the weight of *(all*-*rac)*-α-tocopheryl acetate is achieved.

Furthermore, it has been found that also after long-term-storage of the protected tocopherol of formula (I) of several months (up to 9 months) at a temperature of 25°C or 40°C the amount of tocopherol of formula (II) remains at this low level and particularly does not increase.

Finally, it is also important to stress that this method does not need any additive which needs to remain continuously in the protected tocopherol of formula (I) which eventually would restrict the possible fields of the protected tocopherols because of toxicological doubts or limitations of such an additive. The current method, however, has no such limitations and is therefore optimal for using tocopherols in pharma or food or feed applications.

Particularly surprisingly, it has been found that the amount of tocopherol of formula (II) in the protected tocopherol of formula (I) is significantly reduced in cases where the protected tocopherol of formula (I) is submitted to thermal stress, particularly if the protected tocopherol of formula (I) is rectified. By using the method of the invention, it is particularly possible to obtain highly purified protected tocopherols, especially pharma grade protected tocopherols, of formula (I) with a reduced amount of tocopherols of formula (II).

Therefore, in a preferred embodiment a protected tocopherol of formula (I), particularly (*all*-*rac*)-α-tocopheryl acetate, with a content of more than 96.5 % by weight, particularly more than 97.5 % by weight) of said protected tocopherol of formula (I) and an amount of less than 0.5 % by weight, particularly less than 0.25 % by weight, of tocopherol of formula (II), particularly (*all*-*rac*)-α-tocopherol, can be obtained.

In a further aspect the invention relates to the use of an acid activated bleaching earth for the purification of tocopherol of formula (II) in the form of neat tocopherol of formula (II), or in the form of a solution comprising more than 60 % by weight of tocopherol of formula (II). The tocopherol of formula (II) has been already described above in detail.

The protected tocopherol of formula (I) can be used in different fields. Preferably it can be used in the field of pharma, food or feed.

Therefore, particularly the process of preparing a food or feed composition with a reduced content of tocopherol of formula (II) comprising the steps of
i) preparing a protected tocopherol of formula (I) with a reduced content of tocopherol of formula (II) according to the method as described above;
   and
ii) adding the protected tocopherol of formula (I) with a reduced content of tocopherol of formula (II) yielding from step i) to at least one dietary supplement or food or feed ingredient;
represents a further aspect of the present inventions.

Therefore, particularly the process of preparing a pharmaceutical composition with a reduced content of tocopherol of formula (II) comprising the steps of
i) preparing a protected tocopherol of formula (I) with a reduced content of tocopherol of formula (II) according to the method as described above;
   and
ii') adding the protected tocopherol of formula (I) with a reduced content of tocopherol of formula (II) yielding from step i) to at least one pharmaceutical ingredient;
represents a further aspect of the present invention.

It is not understood, and hence, it is very surprising that the contacting of the tocopherol of formula (II) prior to the protection reaction in the formation of protected tocopherol of formula (I), with an acid activated bleaching earth leads to a reduced content of tocopherol of formula (II) in protected tocopherol of formula (I), particularly after thermal stress as occurring during rectification of protected tocopherol of formula (I).

### Examples

### Comparative example Ref. 1:

In a cascade of 3 stirred vessels a stream of 100 kg/h of (*all*-*rac*)-α-tocopherol was fed and stirred at 140 °C and at an absolute pressure of 50 mbar. The mean residence time in the 3-reactor cascade was 90 minutes.

100 kg of the so treated (*all*-*rac*)-α-tocopherol was rectified in a distillation column and subsequently esterified by adding 72.3 kg acetic anhydride and 0.65 kg pyridine at total reflux (atmospheric pressure) to form (*all*-*rac*)-α-tocopheryl acetate. After distilling off the excess of acetic anhydride, pyridine, and the acetic acid, and a further rectification (sump temperature = 275°C) to obtain highly purified (*all*-*rac*)-α-tocopheryl acetate. The content of (*all*-*rac*)-α-tocopherol in the comparative (*all*-*rac*)-*α*-*tocopheryl acetate **Ref. 1**,* obtained by this manner, was measured using the method indicated below.

### Example 1

In the same cascade of 3 stirred vessels, as used for the comparative example ***Ref.1,*** to a stream of 100 kg/h of (*all*-*rac*)-α-tocopherol, as used for comparative example ***Ref*.*1*,** continuously 1.6 kg/h of TONSIL® SUPREME 115 FF was added and stirred at 140 °C and at an absolute pressure of 50 mbar. At the exit the acid activated bleaching earth was continuously filtered. The mean residence time for the contacting in the same cascade of 3 stirred vessels was 90 minutes.

100 kg of the so purified (*all*-*rac*)-α-tocopherol was rectified in a column, esterified and rectified identically to the comparative example ***Ref.1*** to yield highly purified (*all*-*rac*)-α-tocopheryl acetate. Except the contacting with the acid activated bleaching earth, the example ***1,*** hence, has been prepared identically as the comparative example ***Ref. 1.***

The content of (*all*-*rac*)-α-tocopherol in the example ***1,*** obtained by this manner, was measured using the method indicated below.

### Quantification of (all-rac)-αin (all-rac)-α-tocopheryl acetate

The amount (*all*-*rac*)-α-tocopherol is measured by means of a gas chromatograph (Agilent, model 6850) using an autosampler, a FID detector and a column HP Ultra 2 (Crosslinked 5 % phenylsilanol, 95 % methylsilanol; length 25 m, 0.32 mm internal diameter, 0.52 µm film) and the following parameters: carrier gas: H₂, constant flow: 2.5ml/min, injector temperature: 280°C, injection volume: 1.0 µl, temperature program: 180°C to 280°C rate 20.0 °C/min), detector temperature: 300°C). The sample was injected as a solution of 4 mg/ml in heptane. The peak maximum of (*all*-*rac*)-α-tocopherol was detected a retention time of 13.1 minutes. The amount was determined by calibration with an authentic reference substance of (*all*-*rac*)-α-tocopherol.

### Storage of samples Ref. 1 and 1 & Results:

A sample of 2 liters each ***Ref***.***1*** and ***1*** has been taken and filled under argon into separate flasks and sealed under argon. An analytical sample has been taken from the 2 liter samples before filling and the amount of (*all*-*rac*)-α-tocopherol therein has been determined (storage time: 0 month).

Half of the flasks containing ***Ref***.***1* or *1*** have been stored at 25°C and 65 % relative humidity, and the other half at 40°C and 75 % relative humidity. After 1 month, 3 months, 6 months and 9 months of storage under these conditions, one flask each was taken out of the storage and an analytical sample has been taken and the amount of therein has been determined accordingly. The values obtained are given in tables 1 and 2.

**Table 1. Amounts of (all-rac)-a-tocopherol during long term storage at 25°C.**

| | Content of (*all*-*rac*)-α-tocopherol [% by weight] in the sample after storage at 25 °C / 65 % relative humidity after | | | | |
|---|---|---|---|---|---|
| | 0 month | 1 month | 3 months | 6 months | 9 months |
| ***Ref***.***1***¹ | 0.40 | n.d.³ | 0.30 | 0.30 | 0.30 |
| ***1***² | 0.20 | n.d.³ | 0.20 | 0.18 | 0.20 |
| Reduction by | 50% | n.d.³ | 33% | 40% | 33% |

| | | | | | |
|---|---|---|---|---|---|
| ¹ Content of (*all*-*rac*)-α-tocopheryl acetate: 98.5 % by weight (determined according to European Pharmacopoeia, 5th edition, 2005, Supplement 5.3, page 3632 - 3633) ² Content of (*all*-*rac*)-α-tocopheryl acetate: 98.8 % by weight (determined according to European Pharmacopoeia, 5th edition, 2005, Supplement 5.3, page 3632 - 3633) ³ n.d.= not determined | | | | | |

**Table 2. Amounts of (all-rac)-α-tocopherol during long term storage at 40°C**

| | Content of (*all*-*rac*)-α-tocopherol [% by weight] in the sample after storage at 40°C / 75 % relative humidity after | | | | |
|---|---|---|---|---|---|
| | 0 month | 1 month | 3 months | 6 months | 9 months |
| ***Ref.1***¹ | 0.40 | 0.30 | 0.30 | 0.30 | 0.30 |
| ***1***² | 0.20 | 0.20 | 0.18 | 0.20 | 0.20 |
| Reduction by | 50% | 33% | 40% | 33% | 33% |

| | | | | | |
|---|---|---|---|---|---|
| ¹ Content of (*all*-*rac*)-α-tocopheryl acetate: 98.5 % by weight (determined according to European Pharmacopoeia, 5th edition, 2005, Supplement 5.3, page 3632 - 3633) ² Content of (*all*-*rac*)-α-tocopheryl acetate: 98.8 % by weight (determined according to European Pharmacopoeia, 5th edition, 2005, Supplement 5.3, page 3632 - 3633) . | | | | | |

The results of table 1 and 2 show that an (*all*-*rac*)-α-tocopheryl acetate with a strongly reduced amount (*all*-*rac*)-α-tocopherol can be obtained by the method of invention.

## Claims

1. Method of preparing a protected tocopherol of formula (I) with a reduced content of tocopherol of formula (II) comprising
the steps of
a) contacting tocopherol of formula (II), in the form of neat tocopherol of formula (II) or in the form of a solution comprising more than 60 % by weight of tocopherol of formula (II), with an acid activated bleaching earth;
and
b) reacting tocopherol of formula (II) after contacting step a) with a protecting agent to yield protected tocopherol of formula (I); 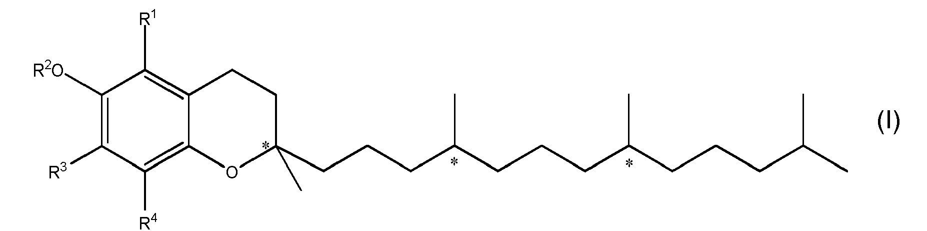 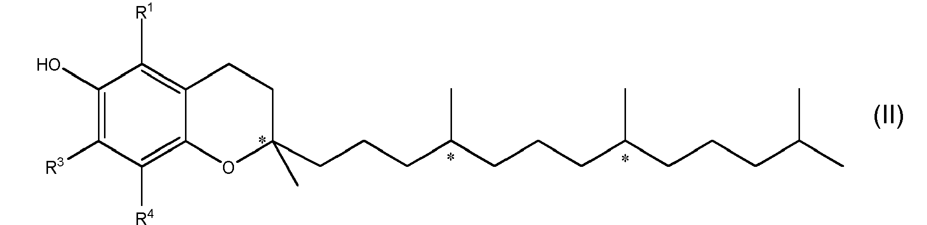
wherein R¹, R³ and R⁴ are independently from each other hydrogen or methyl groups;
R² represents a phenol protection group;
and wherein each * represents an individual chiral centre
**characterized in that**
after the step b) the protected tocopherol is rectified at temperatures measured in the sump in the range of 220-280°C;
and **in that**
the reduction of tocopherol of formula (II) is at least 30 % by weight compared to the amount of the corresponding tocopherol of formula (II) measured in protected tocopherol of formula (I) for the preparation of which no step a) of contacting with acid activated bleaching earth has been used;
and **in that**
the phenol protection group R² forms with the rest of the molecule a chemical functionality which is selected from the group consisting of ester, ether or acetal.

2. Method according to claim 1 **characterized in that** the acid activated bleaching earth is an acid activated bentonite.

3. Method according to anyone of the preceding claims **characterized in that** the acid activated bleaching earth has a specific surface area (BET) between 150 and 400 m²/g, preferably between 250 and 375 m²/g, more preferably between 300 and 350 m²/g.

4. Method according to anyone of the preceding claims **characterized in that** the protected tocopherol of formula (I) is selected from the group consisting of α-tocopheryl acetate, β-tocopheryl acetate, γ -tocopheryl acetate, δ-tocopheryl acetate, particularly α-tocopheryl acetate.

5. Method according to anyone of the preceding claims **characterized in that** the contacting is done at a temperature of between 80 and 160 °C, preferably between 100 and 150°C.

6. Method according to anyone of the preceding claims **characterized in that** the contacting is realized by
adding in step a) the acid activated bleaching earth to the tocopherol of formula (II) and by stirring.

7. Method according to anyone of the preceding claims **characterized in that** weight ratio of acid activated bleaching earth to tocopherol of formula (II) in step a), is between 0.5 / 100 and 5 / 100, particularly is between 1 / 100 and 3 / 100, preferably between 1 / 100 and 2 / 100.

8. Method according to anyone of the preceding claims **characterized in that** the tocopherol of formula (II) in the form of neat tocopherol of formula (II) does not comprise any triglycerides or any fatty acids.

9. Use of an acid activated bleaching earth for the purification of tocopherol of formula (II) in the form of neat tocopherol of formula (II), or in the form of a solution comprising more than 60 % by weight of tocopherol of formula (II) 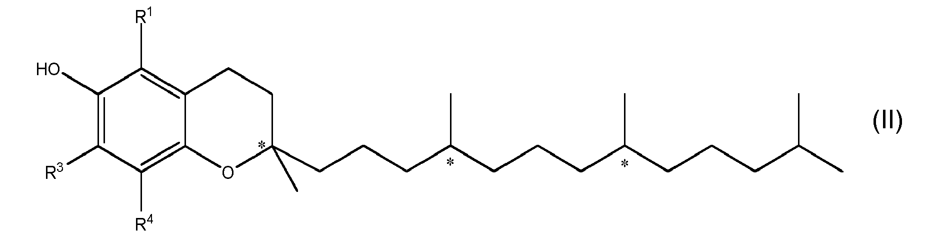
wherein R¹, R³ and R⁴ are independently from each other hydrogen or methyl groups;
R² represents hydrogen or a phenol protection group;
and wherein each * represents an individual chiral centre.

10. Process of preparing a food or feed composition with a reduced content of tocopherol of formula (II) comprising the steps of
i) preparing a protected tocopherol of formula (I) with a reduced content of tocopherol of formula (II) according to the method of claims 1 to 8;
and
ii) adding the protected tocopherol of formula (I) with a reduced content of tocopherol of formula (II) yielding from step i) to at least one dietary supplement or food or feed ingredient;

11. Process of preparing a pharmaceutical composition with a reduced content of tocopherol of formula (II) comprising the steps of
i) preparing a protected tocopherol of formula (I) with a reduced content of tocopherol of formula (II) according to the method of claims 1 to 8;
and
ii') adding the protected tocopherol of formula (I) with a reduced content of tocopherol of formula (II) yielding from step i) to at least one pharmaceutical ingredient.

## Patentansprüche

1. Verfahren zur Herstellung eines Tocopherols der Formel (I) mit einem verminderten Gehalt an Tocopherol der Formel (II), umfassend die folgenden Schritte:
a) Inkontaktbringen von Tocopherol der Formel (II), in Form von Tocopherol als Substanz der Formel (II) oder in Form einer Lösung, umfassend mehr als 60 Gew.-% Tocopherol der Formel (II), mit einer säureaktivierten Bleicherde;
und
b) Umsetzen von Tocopherol der Formel (II) nach dem Schritt des Inkontaktbringens a) mit einem Schutzmittel, wodurch man zu geschütztem Tocopherol der Formel (I) gelangt; 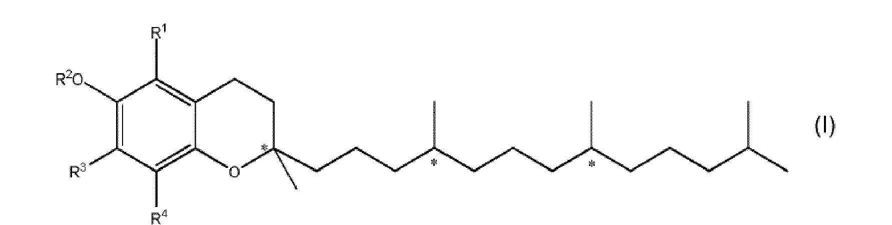 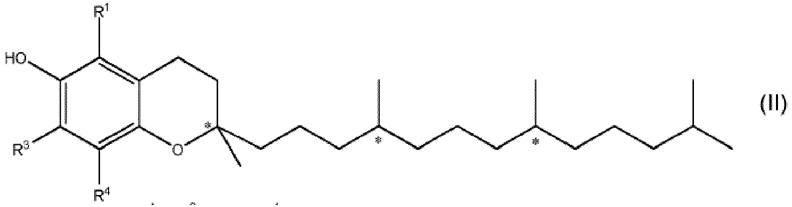
wobei R¹, R³ und R⁴ unabhängig voneinander Wasserstoff- oder Methylgruppen bedeuten;
R² eine Phenolschutzgruppe bedeutet;
und wobei jedes * ein einzelnes Chiralitätszentrum bedeutet,
**dadurch gekennzeichnet, dass**
nach Schritt b) das geschützte Tocopherol bei im Sumpf gemessenen Temperaturen im Bereich von 220-280°C rektifiziert wird;
und dass
die Verminderung an Tocopherol der Formel (II) mindestens 30 Gew.-% im Vergleich zu der Menge des entsprechenden Tocopherols der Formel (II), die in geschütztem Tocopherol der Formel (I) für die Herstellung, für die kein Schritt a) des Inkontaktbringens mit säureaktivierter Bleicherde verwendet worden ist, gemessen wurde, beträgt;
und dass
die Phenolschutzgruppe R² mit dem Rest des Moleküls eine chemische Funktionalität bildet, die aus der Gruppe bestehend aus Ester, Ether oder Acetal ausgewählt ist.

2. Verfahren nach Anspruch 1, **dadurch gekennzeichnet, dass** es sich bei der säureaktivierten Bleicherde um einen säureaktivierten Bentonit handelt.

3. Verfahren nach einem der vorhergehenden Ansprüche, **dadurch gekennzeichnet, dass** die säureaktivierte Bleicherde eine spezifische Oberfläche (BET) zwischen 150 und 400 m²/g, vorzugsweise zwischen 250 und 375 m²/g, stärker bevorzugt zwischen 300 und 350 m²/g, aufweist.

4. Verfahren nach einem der vorhergehenden Ansprüche, **dadurch gekennzeichnet, dass** das geschützte Tocopherol der Formel (I) aus der Gruppe bestehend aus α-Tocopherylacetat, β-Tocopherylacetat, γ-Tocopherylacetat, δ-Tocopherylacetat, insbesondere α-Tocopherylacetat, ausgewählt ist.

5. Verfahren nach einem der vorhergehenden Ansprüche, **dadurch gekennzeichnet, dass** das Inkontaktbringen bei einer Temperatur zwischen 80 und 160°C, vorzugsweise zwischen 100 und 150°C, erfolgt.

6. Verfahren nach einem der vorhergehenden Ansprüche, **dadurch gekennzeichnet, dass** das Inkontaktbringen durch Zusetzen der säureaktivierten Bleicherde zu dem Tocopherol der Formel (II) in Schritt a) und durch Rühren erfolgt.

7. Verfahren nach einem der vorhergehenden Ansprüche, **dadurch gekennzeichnet, dass** das Gewichtsverhältnis von säureaktivierter Bleicherde zu Tocopherol der Formel (II) im Schritt a) zwischen 0,5/100 und 5/100, insbesondere zwischen 1/100 und 3/100, vorzugsweise zwischen 1/100 und 2/100, beträgt.

8. Verfahren nach einem der vorhergehenden Ansprüche, **dadurch gekennzeichnet, dass** das Tocopherol der Formel (II) in Form von Tocopherol der Formel (II) als Substanz keine Triglyceride oder Fettsäuren umfasst.

9. Verwendung einer säureaktivierten Bleicherde zum Reinigen von Tocopherol der Formel (II) in Form von Tocopherol der Formel (II) als Substanz, oder in Form einer Lösung, umfassend mehr als 60 Gew.-% Tocopherol der Formel (II), 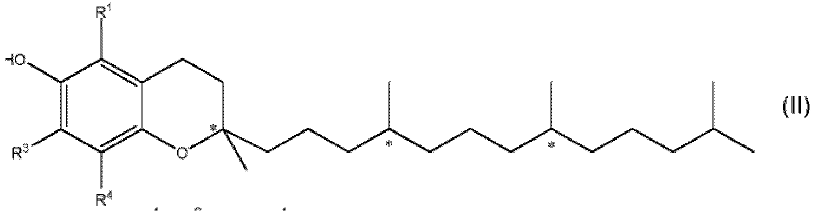
wobei R¹, R³ und R⁴ unabhängig voneinander Wasserstoff- oder Methylgruppen bedeuten;
R² Wasserstoff oder eine Phenolschutzgruppe bedeutet;
und wobei jedes * ein einzelnes Chiralitätszentrum bedeutet.

10. Verfahren zum Herstellen einer Nahrungsmittel- oder Futterzusammensetzung mit einem verminderten Gehalt an Tocopherol der Formel (II), umfassend die folgenden Schritte:
i) Herstellen eines geschützten Tocopherols der Formel (I) mit einem verminderten Gehalt von Tocopherol der Formel (II) nach dem Verfahren der Ansprüche 1 bis 8;
und
ii) Zusetzen des im Schritt i) erhaltenen geschützten Tocopherols der Formel (I) mit einem verminderten Gehalt an Tocopherol der Formel (II) zu mindestens einem Nahrungsmittelzusatzstoff oder Nahrungsmittel- oder Futterbestandteil;

11. Verfahren zur Herstellung einer pharmazeutischen Zusammensetzung mit einem verminderten Gehalt an Tocopherol der Formel (II), umfassend die folgenden Schritte:
i) Herstellen eines geschützten Tocopherols der Formel (I) mit einem verminderten Gehalt von Tocopherol der Formel (II) nach dem Verfahren der Ansprüche 1 bis 8;
und
ii) Zusetzen des im Schritt i) erhaltenen geschützten Tocopherols der Formel (I) mit einem verminderten Gehalt an Tocopherol der Formel (II) zu mindestens einem pharmazeutischen Bestandteil.

## Revendications

1. Méthode de préparation d'un tocophérol protégé de formule (I) ayant une teneur réduite en tocophérol de formule (II), comprenant les étapes consistant à a) mettre en contact du tocophérol de formule (II), sous la forme de tocophérol pur de formule (II) ou sous la forme d'une solution comprenant plus de 60% en poids de tocophérol de formule (II), avec une terre décolorante activée à l'acide ;
et
b) réagir le tocophérol de formule (II) après l'étape de mise en contact a) avec une agent protecteur pour conduire au tocophérol protégé de formule (I) 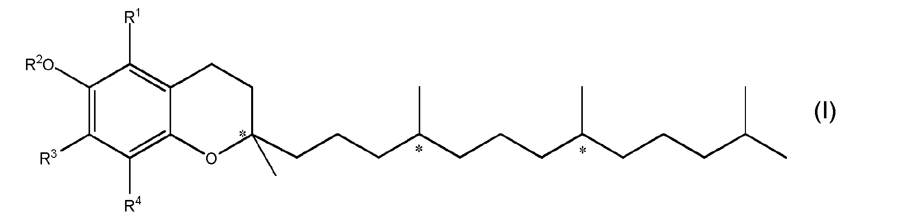 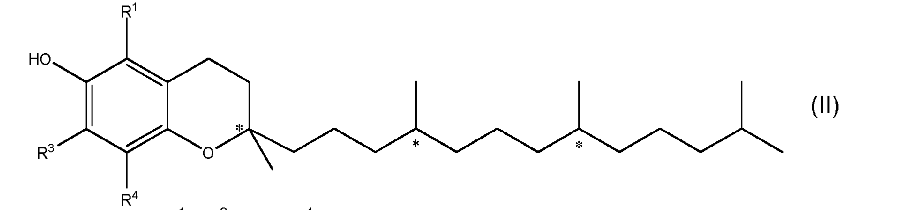
où R¹, R³ et R⁴ sont indépendamment l'un de l'autre hydrogène ou des groupements méthyle ;
R² représente un groupement de protection de phénol ;
et où chaque * représente un centre chiral individuel,
**caractérisée en ce que**
après l'étape b), le tocophérol protégé est rectifié à des températures mesurées dans le puisard dans la plage de 220-280°C ;
et **en ce que**
la réduction du tocophérol de formule (II) est d'au moins 30% en poids par rapport à la quantité de tocophérol de formule (II) correspondant mesurée dans le tocophérol protégé de formule (I) pour la préparation duquel aucune étape a) de mise en contact avec une terre décolorante activée à l'acide n'a été utilisée ;
et **en ce que**
le groupement de protection de phénol R² forme avec le reste de la molécule une fonctionnalité chimique qui est choisie dans le groupe constitué par ester, éther ou acétal.

2. Méthode selon la revendication 1, **caractérisée en ce que** la terre décolorante activée à l'acide est une bentonite activée à l'acide.

3. Méthode selon l'une quelconque des revendications précédentes, **caractérisée en ce que** la terre décolorante activée à l'acide possède une surface spécifique (BET) comprise entre 150 et 400 m²/g, préférablement entre 250 et 375 m²/g, plus préférablement entre 300 et 350 m²/g.

4. Méthode selon l'une quelconque des revendications précédentes, **caractérisée en ce que** le tocophérol protégé de formule (I) est choisi dans le groupe constitué par l'acétate d'α-tocophéryle, l'acétate de β-tocophéryle, l'acétate de γ-tocophéryle, l'acétate de δ-tocophéryle, en particulier l'acétate d'α-tocophéryle.

5. Méthode selon l'une quelconque des revendications précédentes, **caractérisée en ce que** la mise en contact est effectuée à une température comprise entre 80 et 160°C, préférablement entre 100 et 150°C.

6. Méthode selon l'une quelconque des revendications précédentes, **caractérisée en ce que** la mise en contact est réalisée par l'addition dans l'étape a) de la terre décolorante activée à l'acide au tocophérol de formule (II) et par agitation.

7. Méthode selon l'une quelconque des revendications précédentes, **caractérisée en ce que** le rapport pondéral de la terre décolorante activée à l'acide au tocophérol de formule (II) dans l'étape a) est compris entre 0,5/100 et 5/100, particulièrement entre 1/100 et 3/100, préférablement entre 1/100 et 2/100.

8. Méthode selon l'une quelconque des revendications précédentes, **caractérisée en ce que** le tocophérol de formule (II) sous la forme de tocophérol pur de formule (II) ne comprend aucun triglycéride ou aucun acide gras.

9. Utilisation d'une terre décolorante activée à l'acide pour la purification de tocophérol de formule (II), sous la forme de tocophérol pur de formule (II) ou sous la forme d'une solution comprenant plus de 60% en poids de tocophérol de formule (II), 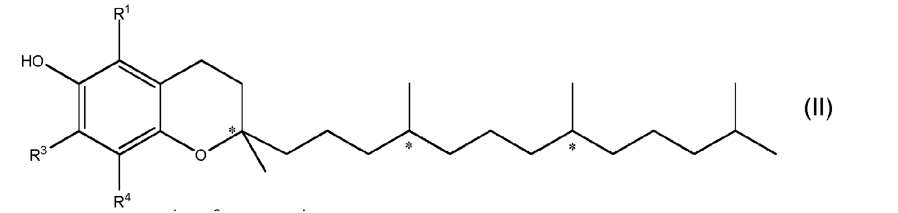
où R¹, R³ et R⁴ sont indépendamment l'un de l'autre hydrogène ou des groupements méthyle ;
R² représente un groupement de protection de phénol ;
et où chaque * représente un centre chiral individuel.

10. Procédé de préparation d'une composition d'aliment ou d'aliment pour animaux ayant une teneur réduite en tocophérol de formule (II), comprenant les étapes consistant à
i) préparer un tocophérol protégé de formule (I) ayant une teneur réduite en tocophérol de formule (II) selon la méthode selon les revendications 1 à 8 ; et
ii) ajouter le tocophérol protégé de formule (I) ayant une teneur réduite en tocophérol de formule (II) issu de l'étape i) à au moins un complément diététique ou ingrédient pour aliment ou aliment pour animaux.

11. Procédé de préparation d'une composition pharmaceutique ayant une teneur réduite en tocophérol de formule (II), comprenant les étapes consistant à i) préparer un tocophérol protégé de formule (I) ayant une teneur réduite en tocophérol de formule (II) selon la méthode selon les revendications 1 à 8 ; et ii') ajouter le tocophérol protégé de formule (I) ayant une teneur réduite en tocophérol de formule (II) issu de l'étape i) à au moins un ingrédient pharmaceutique.
